(19) Europäisches Patentamt
European Patent Office
Office européen des brevets

(11) Veröffentlichungsnummer: **0 038 972**
**A1**

# (12) EUROPÄISCHE PATENTANMELDUNG

(21) Anmeldenummer: **81102692.1**

(22) Anmeldetag: **09.04.81**

(51) Int. Cl.³: **C 07 D 249/08**
C 07 C 131/00, C 07 C 87/68

(30) Priorität: **30.04.80 DE 3016569**

(43) Veröffentlichungstag der Anmeldung:
**04.11.81 Patentblatt 81/44**

(84) Benannte Vertragsstaaten:
**AT BE CH DE FR GB IT LI NL SE**

(71) Anmelder: **BASF Aktiengesellschaft**
**Carl-Bosch-Strasse 38**
**D-6700 Ludwigshafen(DE)**

(72) Erfinder: **Schaefer, Peter, Dr.**
**Im Buegen 16**
**D-6719 Kirchheim(DE)**

(72) Erfinder: **Mangold, Dietrich, Dr.**
**Hermann-Walker-Strasse 49**
**D-6903 Neckargemuend(DE)**

## (54) Verfahren zur Herstellung von Alkylarylketoximethern.

(57) Die vorliegende Erfindung betrifft ein Verfahren zur Herstellung von Alkylarylketoximethern der Formel I

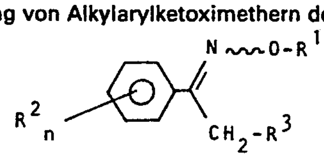

(I)

in welcher
$R^1$ einen organischen Rest,
$R^2$ Wasserstoff oder Halogen,
$R^3$ Alkyl mit 1 bis 5 C-Atomen oder den Rest

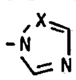

worin X ein Stickstoffatom oder die OH-Gruppe ist,
und
n eine ganze Zahl von 0 bis 3
bedeuten, indem man Alkylralketoxime der Formel II

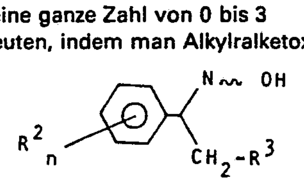

(II)

in welcher $R^2$, $R^3$ und n die oben angegebene Bedeutung haben mit Halogenderivaten der Formel III

$R^1$-Hal (III)

in welcher $R^1$ die oben angegebene Bedeutung hat und Hal für Halogen steht unter Zusatz eines Phasentransferkatalysators bei Temperaturen zwischen – 10°C und + 100°C mit einem Alkalihydroxid umsetzt.

EP 0 038 972 A1

Verfahren zur Herstellung von Alkylarylketoximethern

Die vorliegende Erfindung betrifft ein neues Verfahren zur Herstellung von Alkylarylketoximethern.

Es ist bekannt, daß man Acetophenoximether erhält, wenn man Acetophenonoxime mit einem Metallhydrid, z.B. Natriumhydrid, in das entsprechende Salz überführt und anschließend durch Einwirken von Alkylierungsmitteln die Oximether gewinnt (vgl. z.B. DE-OS 27 23 942).

Es ist ferner bekannt, daß 7-Hydroxiimino-indolizin-Derivate unter Verwendung quartärer Ammoniumhydroxide in Oximether überführt werden können. In einer 1. Stufe wird dabei durch Umsetzung des 7-Hydroxiiminoindolizins mit einer wäßrigen Lösung des quartären Ammoniumhydroxids das Ammoniumsalz des 7-Hydroxiiminoindolizins erzeugt, das dann in einer 2. Stufe mit $\alpha$-Halogencarbonsäureestern umgesetzt wird (BE-PS 818 167).

Dieses Verfahren hat den Nachteil, daß das in der ersten Stufe erhaltene Ammoniumsalz isoliert und dann in der zweiten Stufe in einem anderen Lösungsmittel mit dem $\alpha$-Halogencarbonsäureester zur Umsetzung gebracht wird. Die Aufarbeitung erfolgt durch zeitaufwendige Extraktions- und Waschvorgänge. Hinzu kommt die nicht befriedigende Ausbeute.

Es ist nun bekannt geworden, daß $\alpha$-Azolylacetophenonoximether durch Umsetzung von $\alpha$-Azolylacetophenonoximen mit Halogenderivaten in einem Zweiphasensystem, beispielsweise wäßrige Natronlauge/Toluol, unter Zusatz eines Phasentransferkatalysators erhalten werden können (DE-OS 28 16 817).

Sws/BL

Dieses Verfahren weist jedoch eine Reihe von Nachteilen auf. So wird die Base in einem hohen Überschuß, bezogen auf das α-Triazolylacetophenonoxim eingesetzt. Verbunden mit der Reinigung der Endprodukte, die durch aufeinanderfolgende Waschvorgänge mit Salzlösungen erfolgt, bedingt diese Verfahrensweise eine starke Abwasserbelastung. Der Einsatz großer Mengen Base und die Bewältigung der erheblichen Salzbelastung bei der Aufarbeitung erfordert somit hohe Fertigungskosten, die noch zusätzlich durch die sehr langen Reaktionszeiten und die äußerst niedrigen Ausbeuten erheblich gesteigert werden. Somit ist dieses Verfahren in seiner Gesamtheit sehr unwirtschaftlich.

Es wurde nun gefunden, daß man Alkylarylketoximether der Formel I

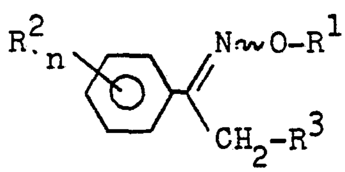

(I)

in welcher

$R^1$ Alkyl, Alkenyl, Alkinyl, Cycloalkyl oder Aralkyl mit jeweils bis zu 20 C-Atomen,

$R^2$ Wasserstoff oder Halogen,

$R^3$ Alkyl mit 1 bis 5 C-Atomen oder den Rest

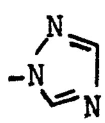

worin X ein Stickstoffatom oder die CH-Gruppe ist, und

n eine ganze Zahl von 0 bis 3

bedeuten, in hoher Ausbeute und Reinheit erhält, wenn man Alkylarylketoxime der Formel II

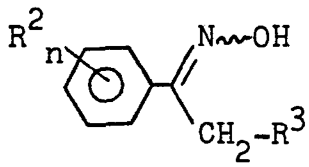

(II)

in welcher $R^2$, $R^3$ und n die oben angegebene Bedeutung haben mit Halogenderivaten der Formel II

$$R^1\text{-Hal} \qquad \text{(III)},$$

in welcher $R^1$ die oben angegebene Bedeutung hat und Hal für Halogen steht, unter Zusatz eines Phasentransferkatalysators bei Temperaturen zwischen $-10^{o}C$ und $+100^{o}C$ mit einem Alkalihydroxid umsetzt.

In einer bevorzugten Durchführungsform des erfindungsgemäßen Verfahrens werden Alkylarylketoxime der Formel II mit einem Überschuß der Halogenderivate der Formel III und festem Alkalihydroxid unter Zusatz eines Phasentransferkatalysators ohne Verwendung eines Lösungsmittels umgesetzt.

Es ist als ausgesprochen überraschend zu bezeichnen, daß bei den erfindungsgemäßen Verfahrensbedingungen die Alkylarylketoximether der Formel (I) in hoher Ausbeute und Reinheit gebildet werden, da bei der Alkylierung von Ketoximen wegen des ambidenten Verhaltens des Ketoximanions in erheblichen Mengen auch N-Alkylierung unter Bildung von Nitronen eintritt (vgl. Houben-Weyl, Methoden der organischen Chemie, Bd. 10/4, Thieme-Verlag, Stuttgart, 1968).

Das erfindungsgemäße Verfahren weist eine Reihe von Vorteilen auf. So wird das Alkalihydroxid in höchstens 50 % Überschuß eingesetzt und bei Verwendung von festem Alkalihydroxid die Hauptmenge des gebildeten Alkalisalzes durch

Filtration abgetrennt, wodurch die Salzbelastung des Abwassers äußerst gering bleibt. Sehr kurze Reaktionszeiten, einfache Aufarbeitung, im allgemeinen keine Rückgewinnung des Lösungsmittels, geringe Abwassermengen und hohe Ausbeuten von 80 % und mehr, machen das erfindungsgemäße Verfahren sehr wirtschaftlich.

Die als Ausgangsprodukte verwendeten Halogenderivate sind durch die Formel III allgemein definiert. In dieser Formel steht Hal vorzugsweise für Chlor und Brom, insbesondere für Chlor.

Als Phasentransferkatalysatoren kommen für das erfindungsgemäße Verfahren quartäre Ammoniumsalze wie N,N-Dibenzyl-N,N-dimethylammoniumchlorid, N-Benzyl-N,N,N-trimethyl-ammoniumchlorid oder N-Dodecyl-N,N,N-trimethylammoniumchlorid sowie quartäre Phosphoniumsalze wie Tetraphenylphosphoniumbromid in Frage. Besonders bevorzugt ist N-Benzyl-N,N,N-triethylammoniumchlorid. Der Katalysator wird in einer Konzentration von 1 bis 25 Mol.%, bezogen auf das Oxim der Formel II eingesetzt. Besonders bevorzugt ist der Konzentrationsbereich 1 bis 5 Mol.%.

Das erfindungsgemäße Verfahren kann beispielsweise in Gegenwart einer 50 %igen wäßrigen Lösung eines Alkalihydroxids oder eines gepulverten Alkalihydroxids durchgeührt werden. Als Alkalihydroxide eignen sich besonders Natrium- und Kaliumhydroxid. Besonders bevorzugt wird Natriumhydroxidpulver. Das Molverhältnis Base zu Oxim der Formel II beträgt 1,0 bis 1,5.

Die Reaktionstemperaturen können in einem größeren Bereich variiert werden. Es ist jedoch zweckmäßig bei Temperaturen unter +50°C zu arbeiten. Bei Verwendung von aktivierten Halogenderivaten der Formel III ist der Temperaturbereich zwischen 0°C und +20°C besonders bevorzugt.

Das erfindungsgemäße Verfahren kann in Gegenwart oder Abwesenheit eines Lösungsmittels durchgeführt werden. Als Lösungsmittel kommen chlorierte, aromatische Kohlenwasserstoffe wie Chlorbenzol, Dichlorbenzol oder Chlortoluol sowie chlorierte aliphatische Kohlenwasserstoffe wie Dichlormethan in Frage. Ist das an der Reaktion beteiligte Halogenderivat der Formel III unter den erfindungsgemäßen Verfahrensbedingungen flüssig, wie Benzylchlorid oder Propargylchlorid, so ist die Verwendung dieses Halogenderivates als Lösungsmittel besonders bevorzugt.

Bei der Durchführung des erfindungsgemäßen Verfahrens setzt man vorzugsweise auf 1 Mol Oxim der Formel II 5 bis 10 Mol des Halogenderivates der Formel III ein und versetzt im Laufe der Reaktion mit vorzugsweise 1,5 Mol Alkalihydroxid.

Zur Isolierung der erfindungsgemäßen Stoffe wird das Reaktionsgemisch filtriert und zur Entfernung wasserlöslicher Feststoffe mit Wasser gerührt. Nach Abtrennen der wäßrigen Schicht wird das überschüssige Halogenderivat und gegebenenfalls das Lösungsmittel durch Destillation entfernt.

Die erfindungsgemäßen Stoffe sind wertvolle Zwischenprodukte zur Herstellung von Pflanzenschutzmittel oder zeichnen sich bekanntermaßen durch sehr gute Wirksamkeit gegen ein breites Spektrum von pflanzenpathogenen Pilzen aus (vgl. DE-OS 27 23 942).

Das erfindungsgemäße Verfahren sei anhand der folgenden Herstellungsbeispiele erläutert:

Beispiel 1

In einem 20 l Kessel mit Umwälzpumpe werden 3,95 kg (14,6 Mol) 2,4-Dichlor-α-(1,2,4-triazolyl-(1))-acetophenon--oxim, 332 g (1,46 Mol) N-Benzyl-N,N,N-triethylammoniumchlorid und 10,9 kg (146 Mol) Propargylchlorid vorgelegt, auf 0°C abgekühlt und eine Lösung von 880 g (22 Mol) Natriumhydroxid in 880 g Wasser so zugetropft, daß +2°C nicht überschritten wird. Nach Beendigung der Zugabe wird 1 Stunde bei 0°C umgepumpt, 10 l Wasser zugesetzt und nochmals 0,25 Stunden umgepumpt. Nach Phasentrennung wird das Progargylchlorid in einem Dünnschichtverdampfer abdestilliert. Der kristalline Rückstand wird im Vakuum bei 50°C von Lösungsmittelresten befreit. Man erhält 4,22 kg (93,5 % d.Th.) 2,4-Dichlor-α-(1,2,4-triazolyl-(1))-aceto-phenon--oxim-O-propargylether vom Fp. 74-83°C, der aus 86 % (Z)-Isomer und 14 % (E)-Isomer besteht.

Beispiel 2

In einem 4 l Kolben mit Rührer werden 542 g (2 Mol) 2,4--Dichlor-α-(1,2,4-triazolyl-(1))-acetophenonoxim, 37,2 g (0,16 Mol) Benzyl-triethyl-ammoniumchlorid und 1,90 kg (15 Mol) Benzylchlorid vorgelegt, auf 0°C abgekühlt und 120 g (3 Mol) Natriumhydroxid zugegeben. Nach Beendigung der Zugabe wird 1 Stunde bei +5°C gerührt, der Niederschlag abgesaugt, das Filtrat 3 mal mit 1 l Wasser gewaschen und über Kaliumcarbonat getrocknet. Nach Abdestillieren von Benzylchlorid erhält man 716 g gelbes Öl, das aus 96 % 2,4-Dichlor-α-(1,2,4-triazolyl-(1))-acetophenon-oxim-O-benzylether besteht. Ausbeute: 95,2 % d.Th.

Beispiel 3

In einem 4 l Kolben werden 1,36 kg (5 Mol) 2,4-Dichlor-α-(1,2,4-triazolyl-(1))-acetophenonoxim, 114 g (0,5 Mol) N-Benzyl-N,N,N-triethylammoniumchlorid, 1,92 kg (25 Mol) Allylchlorid und 1,92 kg Dichlormethan vorgelegt, auf 0°C abgekühlt und eine Lösung von 300 g (7,5 Mol) Natriumhydroxid in 300 g Wasser so zugetropft, daß +5°C nicht überschritten wird. Nach Beendigung der Zugabe wird 1 Stunde gerührt, die organische Phase 3 mal mit je 1 l Wasser gewaschen und über Magnesiumsulfat getrocknet. Nach Abdestillieren von Dichlormethan und Benzylchlorid erhält man 1,34 kg (86,2 % d.Th.) 2,4-Dichlor-α-(1,2,4-triazolyl-(1))-acetophenoxim-O-allylether.

Beispiel 4

In einem 2 l Kolben werden 204 g (1 Mol) 2,4-Dichloracetophenonoxim, 60 g (0,1 Mol) N-Benzyl-N,N,N-triethylammoniumchlorid, 0,95 kg (7,5 Mol) Benzylchlorid und 0,95 kg Dichlormethan vorgelegt, auf 0°C abgekühlt und eine Lösung von 60 g Natriumhydroxid in 60 g Wasser zugetropft. Nach Beendigung der Zugabe wird 4 Stunden bei 40°C gerührt und analog Beispiel 3 aufgearbeitet.
Ausbeute: 268 g (91,1 % d. Th.) 2,4-Dichloracetophenonoxim-O-benzylether.

## Patentansprüche

1. Verfahren zur Herstellung von Alkylarylketoximethern der Formel I

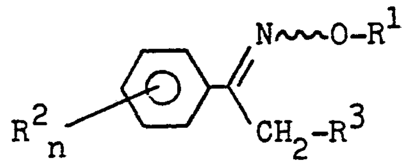

(I)

in welcher

$R^1$ Alkyl, Alkenyl, Alkinyl, Cycloalkyl oder Aralkyl mit jeweils bis zu 20 C-Atomen,

$R^2$ Wasserstoff oder Halogen,

$R^3$ Alkyl mit 1 bis 5 C-Atomen oder den Rest

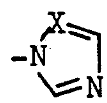

worin X ein Stickstoffatom oder die CH-Gruppe ist, und

n eine ganze Zahl von 0 bis 3

bedeuten, dadurch gekennzeichnet, daß man Alkylarylketoxime der Formel II

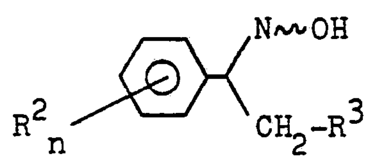

(II)

in welcher $R^2$, $R^3$ und n die oben angegebene Bedeutung haben mit Halogenderivaten der Formel III

$$R^1\text{-Hal} \qquad \text{(III)}$$

in welcher $R^1$ die oben angegebene Bedeutung hat und Hal für Halogen steht unter Zusatz eines Phasentransferkatalysators bei Temperaturen zwischen -10°C und +100°C mit einem Alkalihydroxid umsetzt.

2. Verfahren nach Anspruch 1, dadurch gekennzeichnet, daß als Alkalihydroxid eine 50 %ige wäßrige Natriumhydroxidlösung verwendet wird.

3. Verfahren nach Anspruch 1, dadurch gekennzeichnet, daß als Alkalihydroxid gepulvertes Natriumhydroxid verwendet wird.

4. Verfahren nach Anspruch 1, dadurch gekennzeichnet, daß das Molverhältnis Alkalihydroxid zu Oxim der Formel II 1,0 bis 1,5 beträgt.

5. Verfahren nach Anspruch 1, dadurch gekennzeichnet, daß der Phasentransferkatalysator in einer Konzentration von 1 bis 5 Mol.% bezogen auf das Oxim der Formel II verwendet wird.

6. Verfahren nach Anspruch 1, dadurch gekennzeichnet, daß als Phasentransferkatalysator N-Benzyl-N,N,N-triethylammoniumchlorid verwendet wird.

7. Verfahren nach Anspruch 1 und 3, dadurch gekennzeichnet, daß als Lösungsmittel das Halogenderivat der Formel III verwendet wird.

8. Verfahren nach Anspruch 1 und 3, dadurch gekennzeichnet, daß als Lösungsmittel Dichlormethan verwendet wird.

Europäisches Patentamt

# EUROPÄISCHER RECHERCHENBERICHT

0038972

Nummer der Anmeldung

EP 81 10 2692.1

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D,X | DE - A1 - 2 816 817 (BAYER)<br>* Herstellungsbeispiel 1, Seite 31 *<br>-- | 1,2 |
| D,X | DE - A1 - 2 723 942 (BASF)<br>* Seiten 4 bis 7 *<br>-- | |
| E,X | EP - A1 - 0 028 346 (BAYER)<br>* Seite 14, Zeilen 16 bis 24 *<br>* Einleitung bis Seite 12, Zeile 5 *<br>-- | 1 |
| | ANGEWANDTE CHEMIE, Band 86, Nr. 5, 1974<br>Weinheim<br>DEHMLOW "Phasentransfer-katalysierte Zweiphasenreaktionen in der präparativen organischen Chemie"<br>Seiten 187 bis 196 | |
| | * Seite 187, rechte Spalte * | 6 |
| | * Seite 190, rechte Spalte, unten * | 6 |
| | * Seite 191, rechte Spalte, unten * | 6 |
| | * vollständiges Dokument *<br>---- | 1-8 |

KLASSIFIKATION DER ANMELDUNG (Int. Cl.³)

C 07 D 249/08
C 07 C 131/00
C 07 C 87/68

RECHERCHIERTE SACHGEBIETE (Int Cl.³)

C 07 C 87/00
C 07 C 131/00
C 07 D 249/08

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 10-08-1981 | BREW |